# EUROPÄISCHE PATENTSCHRIFT

(11) **EP 1 009 735 B1**
(45) Veröffentlichungstag und Bekanntmachung des Hinweises auf die Patenterteilung: **06.04.2005**
(21) Anmeldenummer: 98948887.9
(22) Anmeldetag: 26.08.1998
(51) Int. Cl.: C07C 255/46, C07C 253/14, C07C 253/30, C07C 45/71, C07C 41/26, C07C 41/22

(54) **VERFAHREN ZUR HERSTELLUNG VON EINEM 1-(3-CYCLOPENTYLOXY-4-ALKOXYPHENYL)-4-OXOCYCLOHEXANCARBONITRIL**
METHOD FOR PRODUCING A 1-(3-CYCLOPENTENYLOXY- 4-ALKOXYPHENYL)-4- OXOCYCLOHEXANECARBONITRILE
PROCEDE POUR PRODUIRE UN 1-(3-CYCLOPENTYLOXY- 4-ALCOXYPHENYL)-4- OXOCYCLOHEXANECARBONITRILE

(30) Priorität: 28.08.1997 CH 202297
(43) Veröffentlichungstag der Anmeldung: 21.06.2000
(73) Patentinhaber: LONZA AG, CH-3945 Gampel/Wallis (CH)
(72) Erfinder: METTLER, Hans-Peter, CH-3930 Visp (CH)
(74) Vertreter: Waters, David Martin, Dr.
(86) Internationale Anmeldenummer: PCT/EP1998/005504
(87) Internationale Veröffentlichungsnummer: WO 1999/011607

(56) Entgegenhaltungen:
- WO-A-93/19750
- KAZUO HAGA ET AL.: "Condensations of 1,4-Cyclohexanediones and Secondary Aromatic Amines. The Formation of Alkyldiarylamines and Triarylamines" BULLETIN OF THE CHEMICAL SOCIETY OF JAPAN., Bd. 57, Nr. 6, Juni 1984, Seiten 1586-1590, XP002086952 TOKYO JP

## Beschreibung

Die vorliegende Erfindung betrifft ein neues Verfahren zur Herstellung von einem 1-(3-Cyclopentyloxy-4-alkoxyphenyl)-4-oxocyclohexancarbonitril ausgehend von 4-Cyan-4-(3-cyclopentyloxy-4-methoxyphenyl)heptandisäuredialkylester.

1-(3-Cyclopentyloxy-4-alkoxyphenyl)-4-oxocyclohexancarbonitril, im folgenden als CMC abgekürzt, wird zur Herstellung von Pharmazeutika wie zur Herstellung von Phenylcyclohexan-1-ylcarbonsäurederivaten, die die Produktion des Tumor Necrosis Faktors inhibieren, verwendet (WO 95/24381).

Bisher sind mehrere Verfahren zur Herstellung von CMC bekannt. Beispielsweise beschreibt die WO 95/24381 ein 4stufiges Verfahren zur Herstellung von CMC, ausgehend von 3-Cyclopentyloxy-4-methoxybenzaldehyd. Dabei wird 3-Cyclopentyloxy-4-methoxybenzaldehyd in das (3-Cyclopentyloxy-4-methoxyphenylacetonitril überführt, welches dann mit Triton-B und Methylacrylat in den 4-Cyan-4-(3-cyclopentyloxy-4-methoxyphenyl)heptandisäuredimethylester umgesetzt wird. Letzteres wird dann in Gegenwart einer starken Base zum 5-Cyan-5-(3-cyclopentyloxy-4-methoxyphenyl)-2-oxocyclohexancarbonsäuremethylester cyclisiert, welches dann, gelöst in Dimethylsulfoxid, zum CMC decarboxyliert wird. Gemäss dieses Verfahrens wird CMC in einem recht aufwendigen Verfahren mit mehreren Lösungsmittelwechseln in einer Ausbeute von ca. 60 % bez. 4-Cyan-4-(3-cyclopentyloxy-4-methoxyphenyl)heptandisäuredimethylester, welcher im folgenden als CMD abgekürzt wird, erhalten. Nachteilig bei diesem Verfahren ist zum einen die mässige Ausbeute zum anderen das Arbeiten mit Dimethylsulfoxid (DMSO) in einem Bereich, in dem das Lösungsmittel zur thermischen Zersetzung neigt.

Aufgabe der vorliegenden Erfindung war ein einfacheres Verfahren zur Herstellung von CMC bereit zu stellen, bei dem das gewünschte Produkt in guter Ausbeute isoliert wird.

Diese Aufgabe wird mit dem Verfahren gemäss Anspruch 1 gelöst. Erfindungsgemäss wird das Verfahren derart durchgeführt, dass man CMD der allgemeinen Formel 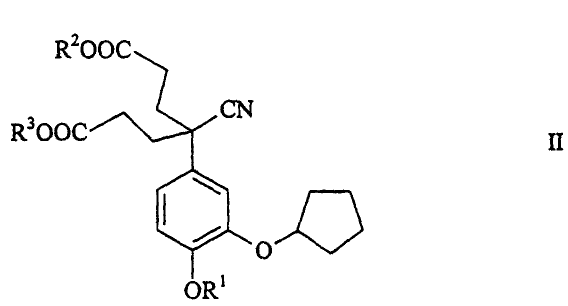 worin R¹, R² und R³ eine C₁₋₅-Alkylgruppe bedeutet und worin R² und R³ die gleiche Bedeutung haben, in Gegenwart einer Base, zu einem 5-Cyan-5-(3-cyclopentyloxy-4-alkoxyphenyl)-2-oxocyclohexancarbonsäurealkylester, im folgenden als CMOM abgekürzt, der allgemeinen Formel 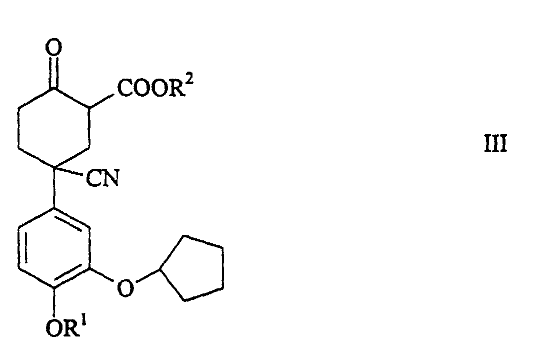 worin R¹ und R² die genannte Bedeutung haben, cyclisiert, die Verbindung der allgemeinen Formel III mit einem Alkalimetallhydrogencarbonat neutralisiert und dieses dann, in Gegenwart von einem Alkalimetallcarbonat, in das Endprodukt gemäss Formel I decarboxyliert.

C₁₋₅-Alkyl kann im folgenden definiert werden als Methyl, Ethyl, Propyl, Isopropyl, Butyl, tert-Butyl und Pentyl.

Als Base kann ein Alkalimetallhydrid oder ein Alkoholat eingesetzt werden. Als Alkoholate können beispielsweise Alkalimetallalkoholate wie Natrium- oder Kaliummethanolat, Natrium- oder Kaliumethanolat, Natrium- oder Kaliumpropanolat oder Natrium- oder Kaliumbutanolat verwendet werden. Als Alkalimetallhydrid kann beispielsweise Natriumhydrid verwendet werden.

Als Alkalimetallhydrogencarbonat kann Natrium- oder Kaliumhydrogencarbonat eingesetzt werden.

Zweckmässig wird die Cyclisierung vom CMD zum CMOM bei einer Temperatur von 20 bis 100 °C, vorzugsweise von 50 bis 70 °C, durchgeführt. Die Decarboxylierung vom CMOM zum CMC wird zweckmässig bei einer Temperatur von 20 bis 100 °C, vorzugsweise von 70 bis 90 °C, durchgeführt.

Die Decarboxylierung wird zweckmässig unter schwach basischen Bedingungen durchgeführt, vorzugsweise zwischen pH 8 und pH 12.

Als Lösungsmittel können für die Cyclisierung und Decarboxylierung polare Lösungsmittel wie beispielsweise Ether eingesetzt werden. Als Ether können Dioxan, Diethylether, Dibutylether, Anisol, Tetrahydrofuran oder Mono-, Di-, Tri- oder Polyethylenglykolether we z. B. 1,2-Dimethoxyethan verwendet werden.

Die Herstellung des Eduktes, das CMD der allgemeinen Formel II, ist im Prinzip aus der PCT WO 93/19 750 bekannt. Zweckmässig wird das CMD der allgemeinen Formel II derart hergestellt, dass man zunächst in einer ersten Stufe ein Isovanillinderivat der allgemeinen Formel 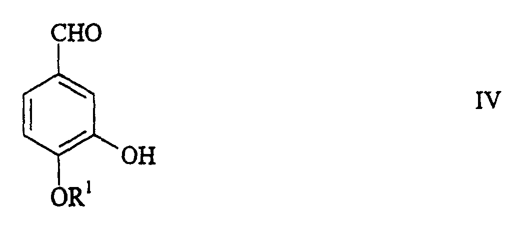 worin R¹ die genannte Bedeutung hat, mit einem Halogencyclopentan in ein 3-Cyclopentyloxy-4-methoxybenzaldehyd der allgemeinen Formel 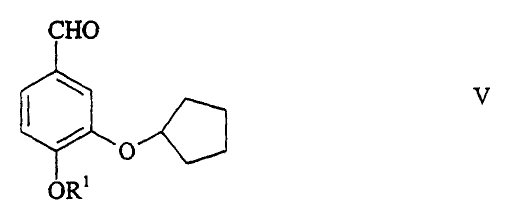 worin R¹ die genannte Bedeutung hat, überführt.

Zweckmässig wird die Umsetzung in der ersten Stufe in Gegenwart eines Alkali- oder Erdalkalimetallcarbonats durchgeführt. Als Alkalimetallcarbonat kann Natrium- oder Kaliumcarbonat und als Erdalkalimetallcarbonat kann Magnesium- oder Calciumcarbonat verwendet werden.

Als Halogencyclopentan kann Fluor-, Chlor-, Brom- oder Jodcyclopentan eingesetzt werden.

Zweckmässig wird die Umsetzung in der ersten Stufe in einem polaren Lösungsmittel durchgeführt. Als polares Lösungsmittel kann beispielsweise Dimethylformamid (DMF) oder ein C₁₋₄-Alkohol wie Methanol, Ethanol, Propanol oder Butanol verwendet werden. Vorzugsweise wird Methanol verwendet.

Die Umsetzung in der ersten Stufe wird zweckmässig bei einer Temperatur von 100 bis 130 °C, vorzugsweise von 110 bis 125 °C, durchgeführt.

In der zweiten Stufe wird der 3-Cyclopentyloxy-4-methoxybenzaldehyd zum (3-Cyclopentyloxy-4-methoxyphenyl)methanol der allgemeinen Formel 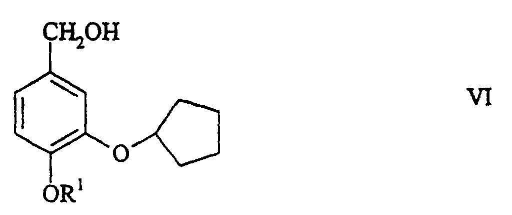 reduziert.

Die Reduktion wird zweckmässig mit Natrium- oder Kaliumborhydrid durchgeführt.

Zweckmässig wird die Reduktion in der zweiten Stufe bei einer Temperatur von -10 bis 90 °C, vorzugsweise von 0 bis 25 °C, durchgeführt.

Üblicherweise wird die Reduktion unter Inertgasatmosphäre durchgeführt.
Als Lösungsmittel können die gleichen wie in der ersten Stufe verwendet werden.

In der dritten Stufe wird das (3-Cyclopentyloxy-4-methoxyphenyl)methanol in ein 4-(Halogenmethyl-2-cyclopentyloxy-1-alkoxy)benzol der Formel 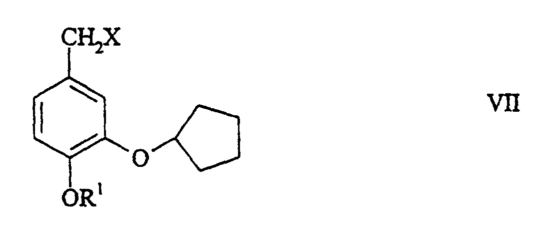 halogeniert.

Als Halogenierungsmittel kann Chlor- oder Bromwasserstoff, insbesondere eine wässrige Lösung von Chlor- oder Bromwasserstoff, verwendet werden. Vorzugsweise wird Chlorwasserstoff verwendet.

Zweckmässig wird die Halogenierung unter Inertgasatmosphäre in einem Kohlenwasserstoff als Lösungsmittel durchgeführt. Als Kohlenwasserstoff kann Toluol, Xylol oder Benzol verwendet werden.

Zweckmässig wird die Halogenierung bei einer Temperatur von 0 bis 50 °C, vorzugsweise von 10 bis 25 °C, durchgeführt.

In der vierten Stufe wird das (Halogenmethyl-2-cyclopentyloxy-1-alkoxy)benzol in ein (3-Cyclopentyloxy-4-alkoxyphenyl)acetonitril der allgemeinen Formel 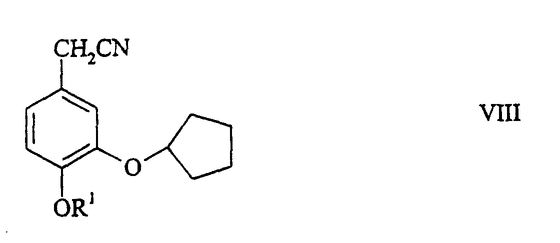 worin R¹ die genannte Bedeutung hat, überführt.

Zweckmässig wird die Umsetzung in der vierten Stufe unter Inertgasatmosphäre und in einem polaren Lösungsmittel durchgeführt. Als polare Lösungsmittel können die gleichen wie in der ersten und in der zweiten Stufe verwendet werden.

Üblicherweise wird die vierte Stufe mit einem Alkalimetallcyanid wie mit Natrium- oder Kaliumcyanid durchgeführt.

Zweckmässig wird die Umsetzung in der vierten Stufe bei einer Temperatur von 10 bis 100 °C, vorzugsweise von 20 bis 50 °C durchgeführt.

In der fünften Stufe wird das (3-Cyclopentyloxy-4-alkoxyphenyl)acetonitril mit einem Alkylacrylat zum CMD der allgemeinen Formel 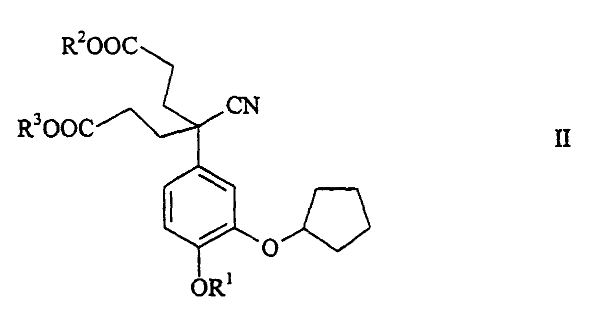 umgesetzt.

Als Alkylacrylat kann Methyl-, Ethyl-, Propyl- oder Butyl-acrylat eingesetzt werden.

Zweckmässig wird die Umsetzung in der fünften Stufe in Gegenwart einer Base wie z. B. in Gegenwart eines nichtionischen Tensids wie Triton-B durchgeführt.

Die Umsetzung in der fünften Stufe kann in einem polaren Lösungsmittel durchgeführt werden. Als polare Lösungsmittel sind geeignet Acetonitril und DMF.

Zweckmässig wird die Umsetzung in der fünften Stufe bei einer Temperatur von 0 bis 60 °C, vorzugsweise von 20 bis 40 °C, durchgeführt.

Das erfindungsgemässe Verfahren zur Herstellung von CMC der allgemeinen Formel I ist aufgrund weniger notwendiger Lösungsmittelwechsel einfach handzuhaben und liefert das gewünschte Produkt in einer Ausbeute zwischen 70 und 80 %.

### Beispiel 1

### Herstellung von (3-Cyclopentyloxy-4-methoxyphenyl)methanol

800 g DMF, 140 g (0,916 mol) Isovanillin, 254 g (1,831 mol) Kaliumcarbonat und 193 g (1,825 mol) Chlorcyclopentan wurden bei Raumtemperatur vorgelegt und auf 120 - 125 °C erhitzt. Nach 3 h wurde auf 25 °C abgekühlt, filtriert und gewaschen mit 508 g Methanol. Das Filtrat wurde abgekühlt auf 0 - 5 °C und in 4 Portionen innert 1 h bei 0 - 15 °C mit 10,8 g Natriumborhydrid versetzt. 30 min nach Ende der Zugabe wurde das Gemisch auf 25 - 30 °C erwärmt und 2 h bei dieser Temperatur gerührt. Man kühlte auf 10 - 15 °C ab und gab innert 20 min 63 g Salzsäure (19,5 %) zu. Dann wurde unter Vakuum der grösste Teil des Lösungsmittels abdestilliert. Zum Rückstand gab man 1160 g Wasser und 1036 g Toluol, rührte 5 min und trennte die untere Wasserphase ab. Man wiederholte die Extraktion zweimal mit total 995 g E-Wasser. Man erhielt 1230,9 g organische Phase (Gehalt 15,61 Gew.% (3-Cyclopentyloxy-4-methoxyphenyl)methanol, GC mit int. Standard).
Ausbeute: 94,4% bez. Isovanillin

### Beispiel 2

### Herstellung von (4-Chlormethyl-2-cyclopentyloxy-1-methoxy)benzol

In dem mit N₂ inertisierten Kolben wurden 327,8 g (3-Cyclopentyloxy-4-methoxyphenyl)methanol-Lösung in Toluol (hergestellt aus 0,356 mol Isovanillin) bei 21,5 °C vorgelegt. Innerhalb von 1 h wurden 105,5 g 32 % HCl (0,926 mol) zugetropft, so dass die Temperatur unter 25 °C blieb. Das Reaktionsgemisch wurde verdünnt mit 110 g Toluol und die Phasen wurden getrennt. Die organische Phase wurde mit 10 g festem NaHCO₃ versetzt, 10 min gerührt und filtriert. Das Filtrat wurde eingeengt. Man erhielt 94,1 g (4-Chlormethyl-2-cyclopentyloxy-1-methoxy)benzol roh als braunes Öl.

### Beispiel 3

### Herstellung von (3-Cyclopentyloxy-3-methoxyphenyl)acetonitril

In dem mit N₂ inertisierten Kolben wurden 85,16 g (4-Chlormethyl-2-cyclopentyloxy-1-methoxy)benzol roh (hergestellt aus 0,364 mol Isovanillin) und 230 g DMF bei 21 °C vorgelegt. Zu der klaren Lösung gab man 10,7 g NaCN. Innert 1 h stieg die Temperatur auf 28,5 °C. Anschliessend wurden nochmals 10,7 g NaCN zugegeben. Das Reaktionsgemisch wurde
1 h weitergerührt und anschliessend innert 30 min. auf 50 °C geheizt. Es wurde noch 1,5 h bei 50 °C gerührt, dann wurde das Lösungsmittel möglichst vollständig abdestilliert. Der Rückstand wurde mit 365 g Toluol und 125 g Wasser extrahiert. Die organische Phase wurde unter Vakuum eingeengt. Man erhielt 78,5 g (3-Cyclopentyloxy-3-methoxyphenyl)acetonitril roh als braunes Öl (Gehalt GC mit internem Standard: 89.3 %).
Ausbeute: 83.3% bez. Isovanillin

### Beispiel 4

### Herstellung von CMD

In dem mit N₂ inertisierten Kolben wurden 82,2 g (3-Cyclopentyloxy-3-methoxyphenyl)acetonitril roh (Gehalt 89,0%, 0,316 mol), 406 g Acetonitril und 1 g Wasser bei 21 °C vorgelegt. In einem Tropftrichter wurde eine Lösung von 94 g Methylacrylat (1,092 mol) in 54,5 g Acetonitril vorbereitet, im anderen Tropftrichter wurde eine Lösung von 12,72 g 35 %-iger methanolischer Benzyltrimethylammoniumhydroxid-Lösung (Triton-B) (ca. 5 % Wasser Gehalt) in 54,5 g Acetonitril vorbereitet. Bei 21 °C wurde 1/4 der Methylacrylat-Lösung zugegeben, anschliessend wurde 1/5 der Triton-B Lösung zugegeben (Exothermie : die Temperatur stiegt sofort auf 38 °C). Abkühlen auf 20 °C innert 15 min., dann wurde wieder 1/4 der Methylacrylat-Lösung zugeben, und 10 min. später 1/5 der Triton-B Lösung zugeben (sofortiger Temperaturanstieg auf 34 °C). Abkühlen auf 20 °C innert 15 min. Nach 10 min. wurde wieder 1/4 der Methylacrylat Lösung zugeben, 10 min. später 1/5 der Triton-B Lösung (Temperaturanstieg auf 25 °C). Abkühlen auf 20 °C innert 10 min. Nach 10 min. wurde der Rest von Methylacrylat zugegeben, 10 min. später wurde wieder 1/5 der Triton-B Lösung, nach 5 min. der Rest von Triton-B zugegeben. Das Reaktionsgemisch wurde noch 1,5 h bei 23 °C gerührt. Die braune Lösung wurde eingeengt. Der Rückstand wurde zwischen 430 g Methylcyclohexan und 56 g Wasser bei 80 °C verteilt. Die organische Phase wurde bei der gleichen Temperatur mit 56 g Wasser nachgewaschen und anschliessend aufkonzentriert. Der Rückstand (klare braune Lösung) wurde unter Rühren auf 0 °C abgekühlt und 1 h bei 0 °C gerührt. Das Produkt wurde abgenutscht und zweimal mit wenig kaltem Methylcyclohexan gewaschen, dann getrocknet. Man erhielt 111,2 g kristallines CMD.
Ausbeute: 87,2 % bez. (3-Cyclopentyloxy-3-methoxyphenyl)acetonitril

### Beispiel 5

### Herstellung von CMOM

33,98 g (82 mmol) CMD (97 %-ig) wurden in 90 g Dioxan gelöst und bei Raumtemperatur mit 18,75 g (104 mmol) Natriummethanolat 30% versetzt. Innert 20 min. wurde auf Rückflusstemperatur aufgeheizt. Nach 30 min. war gemäss DC praktisch alles Edukt umgesetzt und man begann Destillat abzuziehen. Innert 90 min. nahm man total 26,4 g Destillat ab. Die Lösung wurde auf 60 °C abgekühlt, man erhielt eine gut rührbare beige Suspension. Dazu gab man 10,03 g (119 mmol) Natriumbicarbonat und 100 g Wasser. Man erhielt ein Gemisch mit zwei fast klaren Phasen. Dieses wurde direkt in die nächste Stufe geführt.

### Beispiel 6

### Herstellung von CMC

Ca. 253 g Reaktionsgemisch aus der Vorstufe wurde zum Rückfluss erhitzt. Bei einer Badtemperatur von 110 °C und einer Innentemperatur von 87 °C wurde während total 10 h gerührt. Gemäss DC-Kontrolle war der Umsatz zu diesem Zeitpunkt vollständig. Aus dem 2-Phasengemisch wurde total 104 g Destillat abgezogen. Zur, auf 85 - 90 °C abgekühlten, gut rührbaren Suspension gab man ein Gemisch von 124 g (161 ml) Methylcyclohexan und 14 g (16 ml) Ethylacetat. Bei 90 °C Reaktortemperatur und leichtem Rückfluss liess man die klare Wasserphase ab und wusch die gelbliche organische Phase bei der gleichen Temperatur mit 80 g Wasser (in zwei Portionen). Die organische Phase wurde innert 1 h auf 0 °C abgekühlt. Nach 1 h bei 0 °C wurde filtriert, der Rückstand mit 80 ml Methylcyclohexan / Ethylacetat 9 : 1 (w/w, kalt) in zwei Portionen gewaschen und getrocknet (ca. 4 h, 20 mbar, 55 °C). Man erhielt 17,91 g Kristallisat mit einem Gehalt von 98,9 % (HPLC). Eindampfen und Trocknen der Mutterlauge gab 2,99 g Produkt mit einem CMC-Gehalt von 62,7 %.
Ausbeute: 17,91 g Produkt mit einem Gehalt von 98,9 % entsprachen 56,5 mmol = 69 %. Ausbeute bez. eingesetztem CMD (d. h. über beide Stufen). Die totale Ausbeute an gebildetem CMC betrug 77 %.

## Patentansprüche

1. Verfahren zur Herstellung von einem 1-(3-Cyclopentyloxy-4-alkoxyphenyl)-4-oxocyclohexancarbonitril der allgemeinen Formel 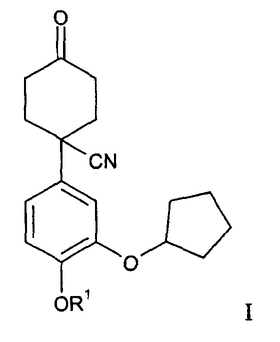 worin R¹ eine C₁₋₅-Alkylgruppe bedeutet, umfassend die Cyclisierung eines 4-Cyan-4-(3-cyclopentyloxy-4-methoxyphenyl)heptandisäuredialkylesters der allgemeinen Formel 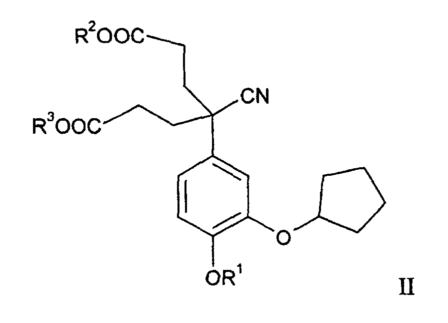 worin R¹ die genannte Bedeutung hat und R² und R³ eine C₁₋₅-Alkylgruppe bedeutet und worin R² und R³ die gleiche Bedeutung haben, in Gegenwart Base, zu einem 5-Cyan-5-(3-cyclopentyloxy-4-alkoxyphenyl)-2-oxocyclohexancarbonsäurealkylester der allgemeinen Formel 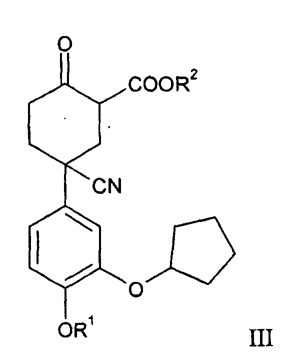 worin R¹ und R² die genannte Bedeutung haben, Neutralisation der Verbindung der allgemeinen Formel III mit einem Alkalimetallhydrogencarbonat und dann Decarboxylierung, in Gegenwart von einem Alkalimetallcarbonat, zum Endprodukt gemäss Formel I.

2. Verfahren nach Anspruch 1, **dadurch gekennzeichnet, dass** man bei der Cyclisierung als Base ein Alkalimetallalkoholat verwendet.

3. Verfahren nach Anspruch 1 oder 2, **dadurch gekennzeichnet, dass** man die Decarboxylierung unter schwach basischen Bedingungen durchführt.

4. Verfahren nach mindestens einem der Ansprüche 1 bis 3, **dadurch gekennzeichnet, dass** man die Cyclisierung und die Decarboxylierung in einem polaren Lösungsmittel durchführt.

5. Verfahren nach Anspruch 4, **dadurch gekennzeichnet, dass** man als polares Lösungsmittel einen Ether verwendet.

6. Verfahren nach Anspruch 1, **dadurch gekennzeichnet, dass** der 4-Cyan-4-(3-cyclopentyloxy-4-methoxyphenyl)heptandisäuredialkylester der allgemeinen Formel II worin R¹, R², und R³ die gennante Bedeutung haben, dadurch hergestellt wird, dass man in einer ersten Stufe ein Isovanilinderivat der allgemeinen Formel 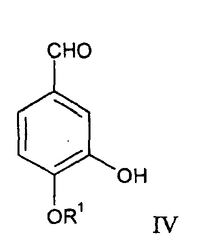 worin R¹ die genannte Bedeutung hat, mit einem Halogencyclopentan in ein 3-Cyclopentyloxy-4-alkoxybenzaldehyd der allgemeinen Formel 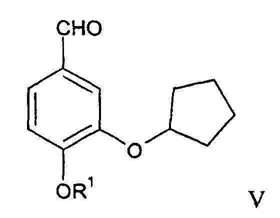 worin R¹ die genannte Bedeutung hat, überführt, diesen in einer zweiten Stufe in ein (3-Cyclopentyloxy-4-alkoxyphenyl)methanol der allgemeinen Formel 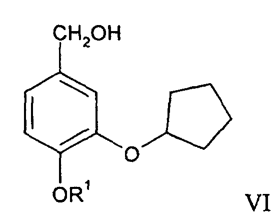 worin R¹ die gennante Bedeutung hat, reduziert, dieses in einer dritten Stufe in ein 4-(Halogenmethyl-2-cyclopentyloxy-1-alkoxy)benzol der allgemeinen Formel 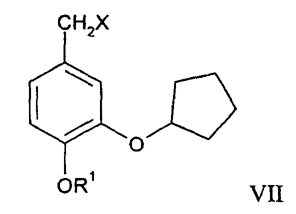 worin R¹ die gennante Bedeutung hat und X ein Halogenatom bedeutet, halogeniert, dieses dann in einer vierten Stufe in ein (3-Cyclopentyloxy-4-alkoxyphenyl)acetonitril der allgemeinen Formel 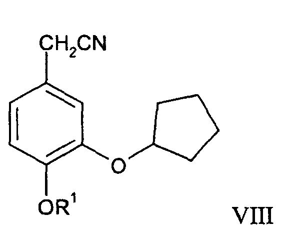 worin R¹ die gennante Bedeutung hat, überführt und letzteres in der fünften Stufe mit einem Alkylacrylat zum 4-Cyan-4-(3-cyclopentyloxy-4-alkoxyphenyl)heptandisäuredialkylester der allgemeinen Formel II umsetzt.

## Claims

1. A process for producing a 1-(3-cyclopentyloxy-4-alkoxyphenyl)-4-oxocyclo-hexanecarbonitrile of the general formula 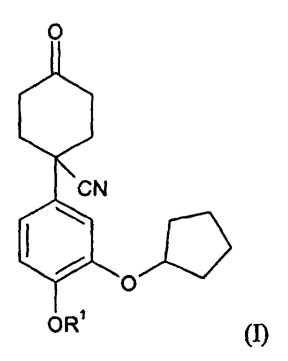 wherein R¹ stands for a C₁₋₅ alkyl group, comprising cyclisation of a 4-cyano-4-(3-cyclopentyloxy-4-methoxyphenyl)heptane diacid dialkyl ester of the general formula 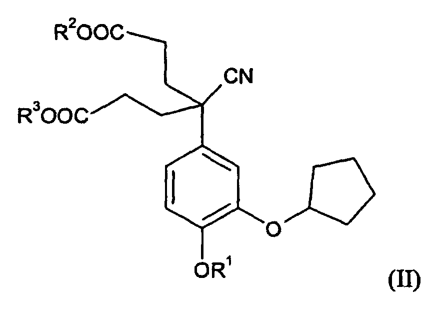 wherein R¹ has the meaning disclosed and R² and R³ stand for a C₁₋₅ alkyl group and wherein R² and R³ have the same meaning, in the presence of a base, to form 5-cyano-5-(3-cyclopentyloxy-4-alkoxyphenyl)-2-oxocyclohexane carboxylic acid alkyl ester of the general formula 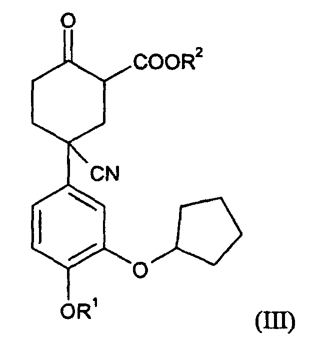 wherein R¹ and R² have the meaning disclosed, neutralisation of the compound of general formula III with an alkali metal hydrogen carbonate and then decarboxylation, in the presence of an alkali metal carbonate, to the end product according to formula I.

2. A process according to claim 1, **characterised in that** an alkali metal alcoholate is used as the base during cyclisation.

3. A process according to claim 1 or 2, **characterised in that** decarboxylation is performed under weakly basic conditions.

4. A process according to at least one of claims 1 to 3, **characterised in that** cyclisation and decarboxylation are conducted in a polar solvent.

5. A process according to claim 4, **characterised in that** an ether is used as polar solvent.

6. A process according to claim 1, **characterised in that** the 4-cyano-4-(3-cyclopentyloxy-4-methoxyphenyl)heptane diacid dialkyl ester of the general formula II wherein R¹, R² and R³ have the meaning disclosed, is produced by, in a first stage, an isovanillin derivative of the general formula 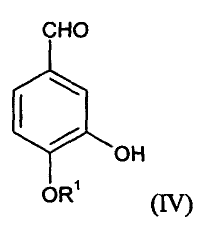 wherein R¹ has the meaning disclosed, being converted with a halogen cyclopentane to a 3-cyclopentyloxy-4-alkoxybenzaldehyde of the general formula 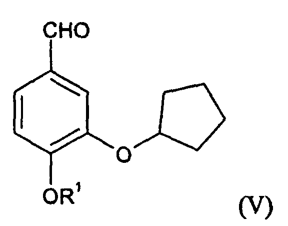 wherein R¹ has the meaning disclosed, this in a second stage being reduced to a (3-cyclopentyloxy-4-alkoxyphenyl)methanol of the general formula 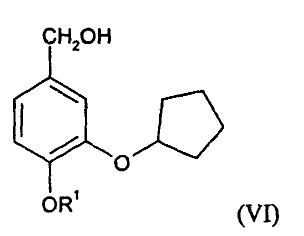 wherein R¹ has the meaning disclosed, this in a third stage being halogenated to a 4-(halogenomethyl-2-cyclopentyloxy-1-alkoxy)benzene of the general formula 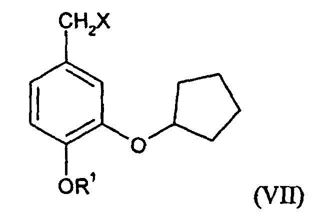 wherein R¹ has the meaning disclosed and X stands for a halogen atom, this then in a fourth stage being converted to a (3-cyclopentyloxy-4-alkoxyphenyl)acetonitrile of the general formula 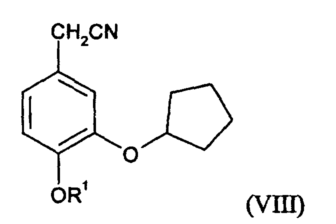 wherein R¹ has the meaning disclosed, and the latter in a fifth stage being reacted with an alkyl acrylate to form 4-cyano-4-(3-cyclopentyloxy-4-alkoxyphenyl)heptane diacid dialkyl ester of the general formula II.

## Revendications

1. Procédé de production d'un 1-(3-cyclopentyloxy-4-alkoxyphényl)-4-oxocyclohexanecarbonitrile de formule générale 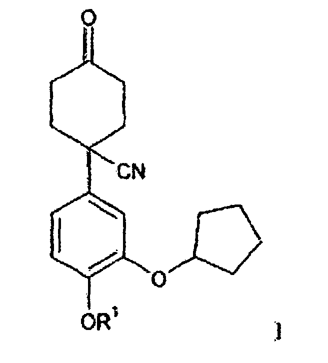 dans laquelle R¹ représente un groupe alkyle en C₁ à C₅, comprenant la cyclisation d'un ester dialkylique d'acide 4-cyano-4-(3-cyclopentyloxy-4-méthoxyphényl)heptanedioïque de formule générale 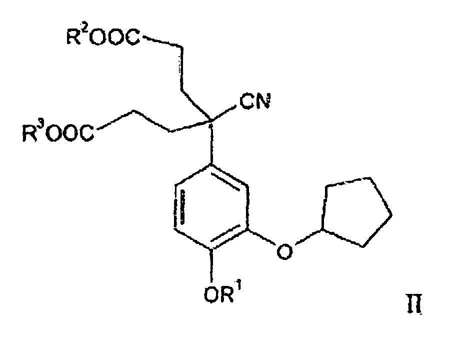 dans laquelle R¹ a la définition mentionnée ci-dessus et R² et R³ représentent un groupe alkyle en C₁ à C₅ et dans laquelle R² et R³ ont la même définition, en présence d'une base, pour former un ester alkylique d'acide 5-cyano-5-(3-cyclopentyloxy-4-alkoxyphényl)-2-oxocyclohexanecarboxylique de formule générale 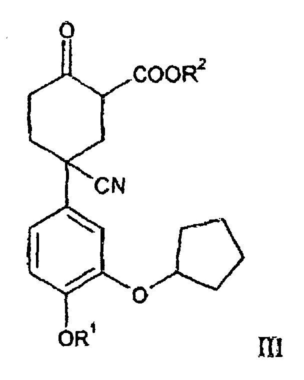 dans laquelle R¹ et R² ont la définition mentionnée ci-dessus, la neutralisation du composé de formule générale III avec un hydrogénocarbonate de métal alcalin puis sa décarboxylation, en présence d'un carbonate de métal alcalin, en produit final selon la formule I.

2. Procédé suivant la revendication 1, **caractérisé en ce qu'**on utilise comme base dans la cyclisation un alcoolate de métal alcalin.

3. Procédé suivant la revendication 1 ou 2,
**caractérisé en ce qu'**on conduit la décarboxylation dans des conditions faiblement basiques.

4. Procédé suivant au moins l'une des revendications 1 à 3, **caractérisé en ce qu'**on conduit la cyclisation et la décarboxylation dans un solvant polaire.

5. Procédé suivant la revendication 4, **caractérisé en ce qu'**on utilise un éther comme solvant polaire.

6. Procédé suivant la revendication 1, **caractérisé en ce qu'**on prépare l'ester dialkylique d'acide 4-cyano-4-(3-cyclopentyloxy-4-méthoxyphényl)heptanedioïque de formule générale II dans laquelle R¹, R² et R³ ont la définition indiquée ci-dessus en transformant dans une première étape un dérivé d'isovanilline de formule générale 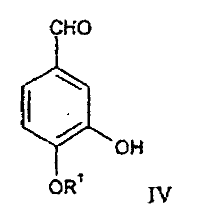 dans laquelle R¹ a la définition indiquée ci-dessus, avec un halogéno-cyclopentane, en un 3-cyclopentyloxy-4-alkoxybenzaldéhyde de formule générale 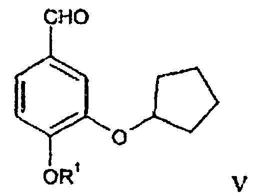 dans laquelle R¹ a la définition indiquée ci-dessus, on réduit ce composé dans une deuxième étape en un (3-cyclopentyloxy-4-alkoxyphényl)méthanol de formule générale 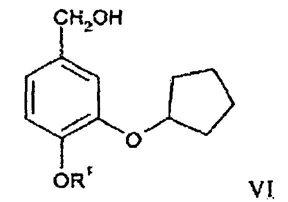 dans laquelle R¹ a la définition mentionnée ci-dessus, on halogène ce composé dans une troisième étape en un 4-(halogénométhyl-2-cyclopentyloxy-1-alkoxy)benzène de formule générale 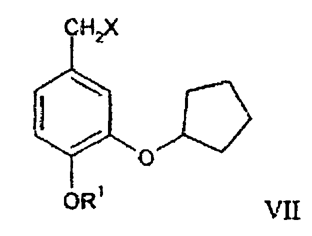 dans laquelle R¹ a la définition mentionnée ci-dessus et X est un atome d'halogène, on transforme ensuite ce composé dans une quatrième étape en un (3-cyclopentyloxy-4-alkoxyphényl)acétonitrile de formule générale 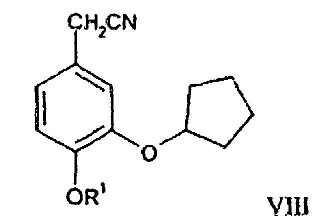 dans laquelle R¹ a la définition mentionnée, et on fait réagir ce dernier composé dans la cinquième étape avec un acrylate alkyle pour former l'ester dialkylique d'acide 4-cyano-4-(3-cyclopentyloxy-4-alkoxyphényl)heptanedioïque de formule générale II.
